# EUROPEAN PATENT SPECIFICATION

(11) **EP 4 149 400 B1**
(45) Date of publication and mention of the grant of the patent: **22.04.2026**
(21) Application number: 21804586.2
(22) Date of filing: 14.05.2021
(51) Int. Cl.: A61F 9/00, A61F 9/007

(54) **METHODS AND COMPOSITIONS FOR REDUCING INTRAOCULAR PRESSURE**
VERFAHREN UND ZUSAMMENSETZUNGEN ZUR SENKUNG DES INTRAOKULAREN DRUCKS
MÉTHODES ET COMPOSITIONS PERMETTANT DE RÉDUIRE LA PRESSION INTRAOCULAIRE

(30) Priority: 15.05.2020 US 202063025384 P
(43) Date of publication of application: 22.03.2023
(73) Proprietor: Georgia Tech Research Corporation, Atlanta, GA 30318 (US)
(72) Inventor: CHAE, Je Min, Lawrenceville, Georgia 30046 (US); ETHIER, Christopher Ross, Atlanta, Georgia 30309 (US); JUNG, Jae Hwan, Cheonan-si, Chungcheongnam-do (KR); PRAUSNITZ, Mark R., Atlanta, Georgia 30307 (US)
(74) Representative: Patentanwaltskanzlei Matschnig & Forsthuber OG
(86) International application number: PCT/US2021/032628
(87) International publication number: WO 2021/231977

(56) References cited:
- US-A1- 2010 247 606
- US-A1- 2014 356 438
- US-A1- 2016 106 587
- US-A1- 2016 166 504
- US-A1- 2018 250 224
- US-A1- 2019 133 933
- US-A1- 2019 133 933
- US-A1- 2020 061 357
- P�REZ LUIS ANDR�S ET AL: "Hyaluronic Acid Hydrogels Crosslinked in Physiological Conditions: Synthesis and Biomedical Applications", BIOMEDICINES, vol. 9, no. 9, 30 August 2021 (2021-08-30), Basel, pages 1113, XP093142931, ISSN: 2227-9059, DOI: 10.3390/biomedicines9091113

## Description

### CROSS REFERENCE TO RELATED APPLICATIONS

This application claims benefit of U.S. Provisional Application No. 63/025,384, filed May 15, 2020.

### STATEMENT REGARDING FEDERALLY SPONSORED RESEARCH OR DEVELOPMENT

This invention was made with government support under grant number R01EY025286 awarded by the National Institutes of Health. The U.S. government has certain rights in this invention.

### BACKGROUND

This invention is generally in the field of ophthalmic therapies, and more particularly to methods and compositions for use in lowering intraocular pressure (IOP), for example, for use in treating glaucoma.

An estimated 75 million people suffer from glaucoma, which is the world's leading cause of irreversible blindness. Vision loss in glaucoma involves the dysfunction and loss of retinal ganglion cell axons and is often associated with elevated intraocular pressure (IOP). In glaucoma, IOP elevation typically occurs due to impeded outflow of aqueous humor from the eye, which drains primarily through the trabecular meshwork located along the outer circumference of the anterior chamber.
Current glaucoma treatments fall into two main categories, both focused on controlling IOP. First is the use of medications (e.g., eye drops) to facilitate the outflow of aqueous humor and/or to decrease the production rate of aqueous humor. However, the need for repeated drug administration results in poor patient adherence that often translates into inadequate IOP control and, thus, disease progression. Furthermore, patients often develop a tolerance for the treatment regimen over time. Moreover, pharmaceutical treatment of glaucoma can have ocular and systemic adverse effects, in addition to suffering from a significant lack of patient adherence. In addition, glaucoma medications often lose efficacy over time so that patients often need to change medications or combine multiple drugs. Moreover, the cost of glaucoma drugs is substantial.

The second approach is surgical, including for example, laser surgery, device implantation, or incisional surgery. However, surgeries are invasive, relatively expensive, and have reduced efficacy over time or upon repeated surgeries. Surgical procedures involve the creation of artificial aqueous humor outflow pathways, either with or without (e.g., trabeculectomy) implantation of a device. In either case, surgical penetration of the globe is required, which is associated with a risk of complications, including hyphema (blood in the anterior chamber), corneal edema (swelling of the cornea), blebitis (infection of a bleb in the eye), endophthalmitis (severe ocular inflammation usually due to infection) and phthisis (shrunken eye with little or no function). Often, scar tissue forms that block the newly created pathways for aqueous humor drainage; this and other complications mean that trabeculectomy has a relatively high failure rate (7% and 15% at 10 and 20 years, respectively), necessitating re-operation. Even with surgery, vision loss progresses within 5 years in 30% of patients. Finally, the surgical treatment of glaucoma is expensive. While surgical implantation of a drainage device usually has a lower complication rate compared to trabeculectomy, it is much more costly and has a failure rate no better than trabeculectomy. Micro-invasive glaucoma surgery may partially alleviate certain shortcomings of glaucoma surgeries by extending the efficacy period and by reducing discomfort, leading to a more rapid recovery. However, such *ab interno* surgical procedures can have serious side effects, such as damage to the corneal endothelium, are costly, and require surgical expertise often not available in developing countries. Hence, there remains a clinical need for a safe and effective option to lower IOP that is drug-free, non-surgical, and provides an extended therapeutic effect, and ideally is relatively easy to administer.

US 2019/133933 A1 discloses injection of a hyaluronic acid gel into the suprachoroidal space to reduce intraocular pressure in a patient. The hyaluronic acid gel is not crosslinked.

US 2016/106587 A1 discloses intraocular administration of a composition that includes a hydrogel and an active pharmaceutical ingredient to reduce intraocular pressure. The composition is placed in the suprachoroidal space of the eye, where the presence of the hydrogel provides sustained release of the drug.

### BRIEF SUMMARY

The scope of the invention is defined by the appended claims.

The references to methods of treatment in the summary and detailed description of the invention are to be interpreted as references to the compositions for use in a method for treatment of the human (or animal) body by therapy.

The invention relates to a composition for use in lowering IOP in a patient according to the appeneded claims. The composition is a biocompatible fluid formulation which is configured, following introduction into the SCS of an eye of the patient, to remain within the SCS for at least 30 days. The biocompatible fluid formulation includes a hydrogel material which is configured to crosslink or further crosslink in vivo. The hydrogel material may include hyaluronic acid and a crosslinker. In still another aspect a hydrogel composition according to the appended claims is provided for use in a method of treating glaucoma.

In a particular example, the method (not being part of the invention) includes (i) inserting a hollow needle into the eye; (ii) injecting a bolus of a composition which contains no active pharmaceutical agent in fluid form through the hollow needle and into the SCS of the eye; and then (iii) then removing the hollow needle from the eye. The needle may be a microneedle. The composition may be a biocompatible fluid formulation which comprises a crosslinked hydrogel, such as one including a crosslinked hyaluronic acid.

In still another aspect, a therapeutic method (not being part of the invention) is provided for a patient indicated to have, or be at risk of developing, glaucoma. In some examples, the method includes injecting into the SCS of an eye of the patient a composition, which contains no active pharmaceutical ingredient and which is effective to maintain a reduction in IOP over an extended period of 30 days or more. In some embodiments, the composition is or includes or turns into a crosslinked hydrogel, such as one including a crosslinked hyaluronic acid.

### BRIEF DESCRIPTION OF THE DRAWINGS

**FIGS. 1A-1D** are graphs which show the effects of a suprachoroidal hydrogel injection on IOP and eye wall thickness in the normotensive rabbit eye, according to embodiments of the present disclosure.
**FIGS. 2A-2D** are graphs which show measurements of aqueous humor outflow facility to identify the mechanism of IOP lowering after injection of *in* situ-forming hydrogel (HA-XL), according to embodiments of the present disclosure.
**FIG. 3** is a graph which shows that a transient increase in IOP was observed after suprachoroidal injection, according to an embodiment of the present disclosure.
**FIGS. 4A-4B** are graphs which show IOP over time before injection, according to embodiments of the present disclosure.
FIGS. 5-8 omitted.
**FIGS. 9A-9D** are graphs which illustrate IOP versus time post-injection using linear regression for single HA compared to Sham, Double HA compared to Sham, and Single HA compared to Double HA, according to an embodiment of the present disclosure.
**FIGS. 10A-10D** are graphs which illustrate IOP post-injection using linear regression and compared to baseline IOP, according to an embodiment of the present disclosure.
**FIG. 11** is a schematic illustration of a hydrogel injection into the suprachoroidal space located between the sclera and the choroid adjacent to the ciliary body, according to an embodiment of the present disclosure.
**FIG. 12** is an image of a microneedle used for injections in the studies described in the examples below.

### DETAILED DESCRIPTION

Methods and compositions have been developed for reducing IOP in patients and maintaining that reduction for an extended period without requiring an active pharmaceutical ingredient or surgical intervention.

The method includes introducing into the suprachoroidal space (SCS) of an eye of the patient a composition effective to maintain a reduction in IOP for at least 30 days. In a preferred embodiment, the composition is introduced by an injection using a hollow microneedle. In some embodiments, the duration of the reduction is at least 60 days, at least 90 days, at least 120 days, at least 180 days, at least 200 days, or at least 220 days. That is, more than 30 to 220 days may elapse before the IOP in the patient would tend to return to an undesirable IOP (baseline) which preceded introduction of the composition. In this way, a maintenance therapy advantageously may be needed only infrequently. For example, the maintenance therapy may involve repeating the introduction of the composition into the SCS of a patient no more than monthly, such as every two months, three months, or four months, or longer.

It has been discovered that this advantageous sustained reduction can be obtained by providing a biocompatible formulation that is both injectable in a fluid form and yet will remain in place in the SCS for an extended period before dissipating. In a preferred embodiment, the composition includes a hydrogel material, which is able to flow through a microneedle and then gel or complete gelation once in the SCS. As used herein, the term "hydrogel material" means a material that is either in hydrogel form or is a material that can become in hydrogel form. In this way, for example, one can inject HA and crosslinker without any crosslinking and consider that a hydrogel material. It was demonstrated that injecting partially crosslinked hydrogel that completes crosslinking in situ in the eye is effective to sustain a reduction in IOP. However, from this discovery, it is envisioned and understood that other materials may be used, including a material that is not crosslinked (i.e., may not yet be considered a hydrogel) before injection and which then crosslinks within the SCS in the eye (i.e., to form a hydrogel). That is, the composition to be injected may be considered a hydrogel, or it be something not considered to be a hydrogel, but will become a hydrogel in the eye.

Advantageously, such an SCS injection of the compositions described herein can be performed as an office procedure, and unlike conventional surgical glaucoma interventions, the injection, for example, with a microneedle, is minimally invasive, which reduces the risk of complications, shortens procedure and recovery time, and reduces cost compared to surgery. The SCS injection of the presently disclosed methods advantageously does not require daily or weekly adherence and does not have the off-target pharmacological effects often associated with drugs.

Furthermore, unlike known implantable devices for promoting aqueous humor outflow, the present methods do not require a device to occupy a space created by damaging tissue to keep open an aqueous humor pathway created by the damage. Instead, the tissue damage is (primarily) by the insertion of a needle (or a microneedle) for the injection into the SCS, which is not the site where the injected formulation is placed, and which is merely a temporary conduit through which the formulation travels in order to reach a natural space in the eye that is expanded by the formulation.

The reduction in IOP is a clinically significant reduction. In some embodiments, the reduction in IOP is at least 1 mm Hg (133.32 Pa). For example, the IOP drop may be at least 2 mmHg, at least 3 mmHg, at least 4 mmHg, at least 5 mmHg, at least 6 mmHg, at least 7 mmHg, at least 8 mmHg, or more. In some embodiments, the reduction may be by an amount up to 30% as compared to IOP without treatment. In a preferred embodiment in humans, the reduction keeps the IOP in the range of 10 to 20 mmHg, and in some embodiments less than 15 mmHg, as measured using means known in the art. The amount of SCS expansion correlates with the amount of IOP reduction, and thus the IOP may be optimized by controlling the amount and/or other characteristics of the composition injected, such as the swelling property of the injected material.

As used herein, the term "suprachoroidal space" or SCS is the potential space between the choroid and sclera, forming part of the uveoscleral pathway. Without being bound to any particular theory, the present methods are believed to reduce IOP by making the SCS wider and thereby providing a larger (more conductive) pathway for aqueous humor flow out of the eye. It may be that the expanding of SCS is directly responsible for reducing IOP by increasing aqueous humor outflow by the unconventional aqueous drainage pathway. It may be that expanding the SCS is indirectly responsible for reducing IOP by increasing aqueous humor outflow through tissues near to the SCS that are expanded or have their geometry altered by the expansion of the SCS. Another possible mechanism is that the expansion of the SCS applies forces on the ciliary body or otherwise affects the ciliary body in a way that causes the ciliary body to decrease the production of aqueous humor.

### The Methods (not being encompassed by the wording of the claims)

The methods described herein are used to lower IOP in a patient. The patient may be a human or other mammal in need of a reduction in intraocular pressure. The patient may be indicated to have, or be at risk of developing, glaucoma.

The methods may be used to treat any ocular disease in which it is advantageous to maintain a lowered IOP. In particular embodiments, the methods are used in the treatment or prophylaxis of glaucoma or ocular hypertension. The methods also could be used in an emergency setting, e.g., to lower an acute IOP spike that occurs in glaucoma, especially angle-closure glaucoma.

By introducing the composition into the SCS, the IOP in the eye is caused to be reduced. More specifically, for up to several hours, the IOP in the eye may increase due to the introduction of the composition into the SCS, but within a few hours, the IOP drops to a level lower than before the introduction. IOP drop may continue for a period of time, e.g., up to a few days, and then reach a maximum IOP drop. Over time, the IOP drop may become smaller, and eventually, there may no longer be a significant IOP drop compared to before the treatment was performed. In some embodiments, the IOP drop steadily decreases over time. In other embodiments, the IOP drop remains relatively constant for a period of time, after which it decreases. In still another embodiment, the IOP drop persists indefinitely.

The method for reducing IOP includes introducing into the SCS of an eye of the patient a composition effective to maintain a reduction in IOP for at least 30 days. The composition generally would be introduced into each eye in need of treatment.

In a preferred embodiment, the introduction of the composition is by injection using a hollow needle or hollow microneedle. In other embodiments, the injection may be performed using multiple needles, such as an array of microneedles. For example, the injection may be done with two to ten needles, e.g., two, three, four or six needles or microneedles, which may be inserted simultaneously to reach different regions of the SCS. Introduction of the composition through a microneedle advantageously facilitates deployment of the composition into the SCS in a minimally invasive manner. Suitable needles and microneedles are known in the art, as are systems and methods for inserting microneedles to access the SCS.

In a preferred embodiment, the method includes (i) inserting a hollow needle (e.g., a microneedle) into the eye; (ii) injecting a bolus of the composition in fluid form through the hollow needle and into the SCS of the eye; and then (iii) removing the hollow needle from the eye.

In embodiments, the volume of the bolus of the composition injected ranges from 10 µl to 400 µl. In some preferred embodiments, the volume ranges from 20 µl to 200 µl, or from 50 µl to 100 µl. In various embodiments, the volume is about 40 µl, about 50 µl, about 60 µl, about 70 µl, about 80 µl, about 90 µl, about 100 µl, about 120 µl, about 140 µl, about 160 µl, or about 180 µl.

In some embodiments, the needle or microneedle is inserted into the eye at a location posterior to the limbus. In some embodiments, the needle or microneedle is inserted at an anterior portion of the SCS, e.g., near the limbus or anterior to the equator of the eye.

For instance, microneedles advantageously may be inserted perpendicularly into the sclera, reaching the SCS in a short penetration distance. In other embodiments, a longer needle or microneedle may be inserted through the sclera at a non-perpendicular angle.

In some embodiments, the reduction in IOP following a single injection is maintained for at least 60 days. In some embodiments, the reduction in IOP following a single injection is maintained for at least 90 days. In some embodiments, the reduction in IOP following a single injection is maintained for at least 120 days. In some embodiments, the reduction in IOP following a single injection is maintained for at least 180 days. In some embodiments, the reduction in IOP following a single injection is maintained for at least 200 days. In some embodiments, the reduction in IOP following a single injection is maintained for at least 220 days. In some embodiments, the reduction in IOP following a single injection is maintained for between 60 and 90 days, between 90 and 120 days, between 120 and 180 days, or between 180 and 220 days. In a preferred embodiment, the reduction in IOP following a single injection is maintained for at least 7 months. For clarity, a "single injection" is a single treatment of injection(s) with one or more needles or microneedles.

In some other embodiments, the injected composition is configured to form a permanent SCS expander, such that the material stays in place in the SCS at least until it is actively removed. In this way, the injected material, which could be a non-degradable gel for example, would be effective to maintain the reduction in IOP for a much longer period, such as for 6 months, 12 months, or multiple years.

To further maintain the reduction of IOP over longer periods, the injection may be repeated periodically. In such a maintenance therapy, the site of follow-on injections may be the same as the initial injection site, or more preferably rotated to a different site in the eye, to reduce any micro-scale trauma (e.g., hemorrhage) and the immune response that causes fibrosis in any particular region of the eye. For example, the injections may be made in a different quadrant of the eye each time, such that the same site is used no more than every fourth injection.

In some alternative embodiments, the composition is introduced into the SCS using a minor surgical incision or by a jet injection or by creating a transscleral pathway using a physical cutting device or other physical or chemical methods. The formulation could be delivered into the SCS by diffusion, convection, or iontophoresis, or other methods known to enhance transport of materials into or across the tissue.

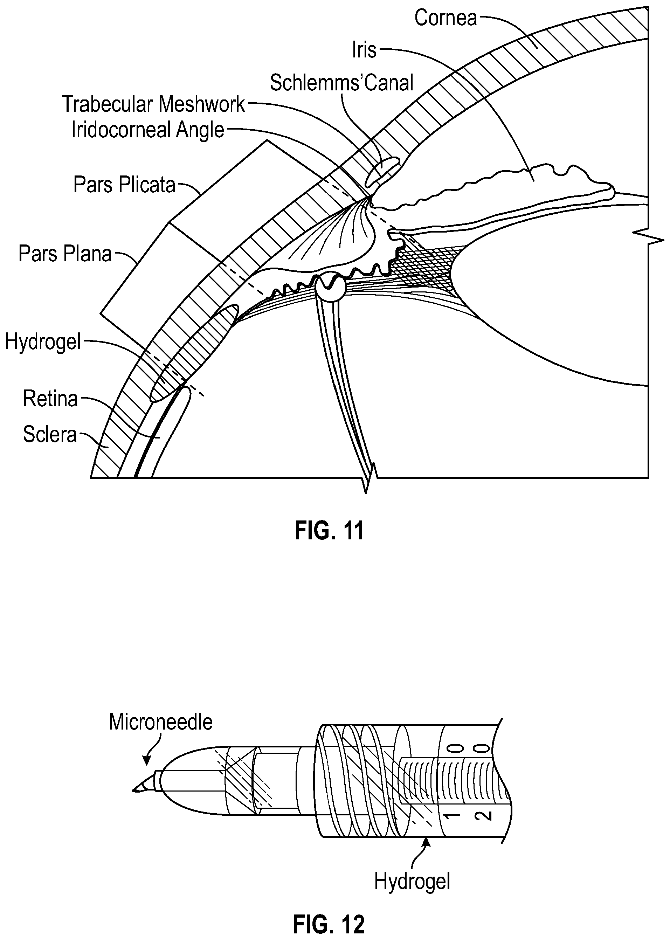

Introduction of the composition into the SCS expands the SCS and maintains that expansion for at least 30 days. **FIG. 11** shows one embodiment of a hydrogel composition within and expanding the SCS. In various embodiments, the SCS is expanded by the composition for a period of at least 60 days, at least 90 days, at least 120 days, at least 180 days, or at least 210 days. The SCS may be expanded by the composition for a period of between 90 and 180 days, or between 180 and 220 days, in some embodiments. In some other embodiments, the SCS remains expanded for longer time periods, for example, using materials that are non-degradable or only very slowly degradable. The degree of the SCS expansion may remain essentially the same over the period, or may gradually decreases over the period, for example if the particular composition is configured to degrade, dissolve, or disentangle and thereby clear from the patient's eye.

In some preferred embodiments, the composition within the SCS biodegrades and is cleared naturally from the patient's eye at a slow enough rate to sustain the IOP reduction for 30 days or more. That is, the composition may dissolve, erode, or degrade by enzymatic hydrolysis into components that can be absorbed into tissues, or dissolved or otherwise carried away by diffusive, convective or other processes in the eye.

In some other embodiments, the composition is essentially non-degradable. In some embodiments, the non-degradable composition remains in the patient's eye indefinitely, until removed surgically, or until its degradation is facilitated. For example, the method could including injecting a second material into the SCS, wherein the second material is configured to dissolve the composition, break bonds in the composition, or otherwise alter the SCS environment around the composition to facilitate its degradation and clearance. For example, the second composition could comprise an enzyme or pH modifier selected for this purpose. In still another example, removal of the non-degradable material may be facilitated by targeted application of energy (e.g., heat, electromagnetic field, ultrasound ) to the site of the gelled/solidified composition to change the properties of the composition in the SCS so that it breaks down and is cleared.

In some embodiments of the methods, the injected composition is a shear-thinning fluid or undergoes *in-situ* gelation or solidification. In preferred methods, the composition is injected as a fluid, which could have dissolved and/or suspended materials in it, and then gels or otherwise solidifies within the SCS.

The solidification or gelation within the SCS may occur by virtue of the selection of an appropriate composition, which itself is configured to gel or solidify following its introduction into the SCS, or which is configured to gel or solidify following interaction with or application of a triggering means to promote the solidification or gelation. For example, gelation triggers may include a change of pH in the SCS environment and/or the composition, a change of temperature, a change of ionic strength or change in the composition. Gelation may occur by contact of the composition with biological fluids within the SCS (e.g., aqueous humor). In some embodiments, the gelling of the composition is facilitated by application of electromagnetic radiation, ultrasound, heat, or other energetic inputs to the composition.

In some embodiments, the composition undergoes a chemical reaction in the SCS. This chemical reaction may be a crosslinking reaction. In some preferred embodiments, the crosslinking may be with covalent bonds, and this will generally provide the longest duration. In other embodiments, the crosslinking may include ionic bonds, hydrogen bonds, hydrophobic interactions, etc.

The composition in the SCS comprises a cross-linked hydrogel. The cross-linked hydrogel may include hyaluronic acid.

In some other embodiments of the methods, the biocompatible materials may not be a gel. Other biocompatible materials may be used so long as the material is flowable at the time of injection and then following injection into the SCS gels, solidifies or otherwise has a change of properties so that it does not flow, or at least flow much more slowly.

In some embodiments of non-hydrogel materials, the composition in the SCS may desirably be porous. For example, the composition may have a nanoscale porosity of a hydrogel or larger microscale pores. Making the pores in the composition may be achieved by including a pore-forming agent dispersed in the composition. The pore forming agent is a sacrificial material that leaves the composition following its injection into the SCS, and thereby leave pores in the regions of the material in which the pore-forming agent previously resided. The pore forming agent may be biocompatible salt particles, for example. In some embodiments, the composition includes a polymer in a solvent with salt particles suspended therein. Upon injection, the solvent diffuses away, causing the polymer to precipitate and form a solid material that has salt particles entrained in it. The salt subsequently dissolves, leaving behind pores. Suitable polymers, solvents, and salts are known in the art.

### The Compositions

The compositions useful in the presently disclosed methods are biocompatible and provided in a fluid form of sufficiently low viscosity that it is suitable for injection through a needle, such as a hollow microneedle.

As used herein, the term "biocompatible" means that the interaction with the body causes acceptable levels of tissue changes, such as local hemorrhage, fibrosis, or other reactions to the formulation, system, and process. Biocompatibility does not mean that there are no changes to the body. Indeed, this invention is designed to make changes, especially to local tissue geometry, that lower IOP.

These compositions may be referred to herein as biocompatible formulations.

The injectable composition may be in the form of an aqueous solution of one or more polymer, monomer or oligomer components, which are selected or adapted to crosslink, gel, or otherwise solidify within the SCS. A gel is considered to be solidified.

The solidified composition expands the SCS. It can do so by expanding the SCS at the time of injection. It may also expand further after the injection, such as by swelling with water from the body. The solidified composition is preferably permeable to water, for example, as a hydrogel or other water-permeable material or as an impermeable material with pores through it that are water-permeable. However, the expansion of the SCS with a solidified composition that is not significantly water-permeable may also reduce IOP. The degree of the SCS expansion may remain the same for a period of time, or it may steadily decrease over time. Additional material may be added at the site of a previously introduced solidified composition, for example, to further expand, or re-expand, the SCS, or at a site that did not previously have a solidified system introduced, for example, to expand a new site in the SCS, for the objective of modifying IOP, for example, to prolong the period of IOP reduction.

The compositions do not need to include an API in order to be effective to reduce IOP. Accordingly, no drug is included in the compositions.

The compositions include one or more materials that following introduction into the SCS will remain in place within the SCS, for example at or near to the site of injection. In embodiments, the materials include one or more biocompatible polymers. For compositions that are degradable, this means that at least a portion of the composition remains within the SCS in an amount effective to maintain the IOP reduction for at least 30 days. In some embodiments, at least a part of the composition remains in the SCS for at least 30 days.

In some embodiments, the composition includes one or more components selected to undergo a chemical reaction in the SCS to increase the viscosity of the composition, such as by gelling or solidification. The chemical reaction may be a crosslinking reaction. In some preferred embodiments, the crosslinking may be with covalent bonds, and this will generally provide the longest duration. In other embodiments, the crosslinking may occur by using other types of bonds, including but not limited to ionic bonds, hydrogen bonds, van der Waals bonds, and hydrophobic interactions.

In some preferred embodiments, the fluid composition comprises a biocompatible hydrogel. The hydrogel may be a hyaluronic acid (HA) based hydrogel, a polyethylene glycol (PEG) based hydrogel, or a combination thereof. The gel may be made of other materials known in the art to make hydrogels suitable for medical or pharmaceutical use, including those based on proteins (e.g., collagen, elastin, fibrin, gelatin, silk fibroin), based on polysaccharides (e.g., glycosaminoglycans, alginate, chitosan), based on synthetic materials (N-isopropylacrylamide, polymethacrylic acid, polyvinyl pyrrolidone, captopril, poly-2-hydroxyethyl methacrylate (HEMA)).

In a preferred embodiment, the gelled composition comprises an elastic, biodegradable and biocompatible hydrogel, as known in the art. It may include natural and/or synthetic polymers, including those derived silk fibronin, PEG, polycaprolactone, or the like.

In some preferred embodiments, the composition includes a co-formulation of a biocompatible hydrogel and a crosslinker. In some embodiments, the crosslinker is a prepolymer solution which will undergo *in-situ* gelation. In some embodiments, the prepolymer solution comprises polyethylene glycol diacrylate (PEGDA) or other crosslinking agents known in the art. In some embodiments, the PEGDA is at least 5% w/v of the hydrogel.

In a preferred embodiment, the composition comprises a cross-linked hyaluronic acid (HA). HA may be used to form the gel because it is naturally found in the eye as a component of vitreous humor and the extracellular matrix of the sclera, the choroid, the cornea, and other tissues. The HA needs to be cross-linked or otherwise transformed in order for the HA hydrogel to remain within the SCS for a sustained period effective to reduce IOP for at least 30 days. Otherwise, a HA hydrogel will be cleared too rapidly.

In some embodiments, the composition includes HA modified to include functional groups that facilitate crosslinking. For example, in some embodiments, the HA comprises added thiol groups. In some preferred embodiments, the composition includes HA co-formulated with a crosslinking agent, such as PEGDA. In some embodiments, the composition comprises PEGDA in an amount between 3% w/v and 15% w/v. In some embodiments, the amount of PEGDA is at least about 5 w/v% of the HA hydrogel.

In some other embodiments, the change from a flowing material during injection to a stationary material after injection can be done using a shear-thinning fluid. It can also be done using in situ gelation triggered by mechanisms such as a change of temperature, pH, tonicity, divalent cation concentration, and other known methods. The increased viscosity or gel formation or solidification (aka solidification) can occur due to physicochemical interactions between formulation components that do not involve the formation of covalent bonds, including phase change. The solidification can involve the formation of covalent bonds between formulation components. One method involves the introduction of a polymer that crosslinks, possibly by the introduction of a crosslinking agent and possibly other agents if needed. It could also involve introducing electromagnetic radiation (UV, visible, IR, or other light; electric and/or magnetic fields) or heat to assist in making crosslinks or otherwise drive the solidification process. It could further involve the delivery of energy by some other modality, such as by ultrasound. The solidification could involve click chemistry. The solidification can involve reaction with the tissue or body fluid. The polymers could be synthetic or naturally occurring, or a combination of both. The solidified system could have a mechanism to break down by hydrolysis, enzymatic reaction, or other causes. In this case, the solidified formulation would not be permanent and would probably eventually be cleared from the injection/solidification site.

In some other embodiments, the compositions could include materials derived from tissues. For example, autologous tissue from the patient could minimize the immune response after implantation with or without chemical, mechanical and enzymatic modifications. For example, autologous fat could be decellularized and modified its natural characteristics with the methods mentioned above.

The present invention may be further understood with reference to the following nonlimiting examples.

### EXAMPLES

Experiments were conducted to evaluate whether compositions could be used to lower IOP when injected into the eye. More specifically, experiments were conducted to evaluate whether microneedles can deliver hydrogel compositions into the SCS, expanding the SCS and lowering IOP for extended periods of time. The examples herein show that that injection of hydrogel into the anterior SCS to expand the SCS of the rabbit eye can reduce IOP without the use of drugs or surgical procedures.

*Microneedle and in situ-forming hydrogel preparation: A* 27-gauge expansion needle was used to fabricate a hollow microneedle. Briefly, after shortening the needle to ~1.0 mm in length using a conventional cutter, the needle was ground with a cordless rotary tool. The length of the needle was measured to be approximately 650-750 µm under a stereomicroscope, and the needle was then sterilized using ethylene oxide. An in situ-forming cross-linked hydrogel (HA-XL) was prepared using 3% (w/v) thiol-modified hyaluronic acid (HA-SH) (Glycosil, ESI Bio, Alameda, CA) and 5% or 9% (w/v) poly(ethylene glycol) diacrylate (PEGDA, MW 3500 Da, JenKem Technology, Beijing, China) dissolved in Hank's Balanced Salt Solution (HBSS). While both the 5% and 9% PEGDA formulations were studied in vitro, only the 9% PEGDA formulation was used in vivo. For the in vivo study, aliquots of HA-SH and PEGDA were prepared in a conventional biosafety cabinet, and sterile HBSS was used to dissolve all compounds. After 15 min at room temperature (20 - 25 °C), the HA-XL hydrogel was loaded in a syringe (1 mL Luer-lock plastic syringe; following aseptic technique, and the microneedle was attached.

*Animals:* Eleven New Zealand White rabbits (either sex, 3.0 - 3.5 kg) were purchased from Charles River Breeding Laboratories (Wilmington, MA). All animal studies were carried out in accordance with the Association for Research in Vision and Ophthalmology (ARVO) Statement for the Use of Animals in Ophthalmic and Visual Research, and all experimental procedures were approved by the Georgia Institute of Technology Institutional Animal Care and Use Committee. After a one week acclimation period, all eyes were confirmed to be clinically normal by an ophthalmic examination before studies were begun.

Three sets of animal studies were performed. In the first, 50 µl of a commercial HA hydrogel (2.4 % (w/v) HA, 6 % cross-linked, Juvederm Ultra XC, Allergan, Irvine, CA) was injected into the SCS in two animals to explore the effects of suprachoroidal expansion on IOP. In each animal, one eye received 50 µl of the commercial HA solution injected in the superotemporal quadrant of the eye, while the other eye received 25 µl of the commercial HA solution injected in the superotemporal quadrant and 25 µl of the commercial HA solution injected in the superonasal quadrant. Both rabbits were euthanized 50 days after the injection.

In the second study, five animals were used to evaluate the safety and efficacy of an *in* situ-forming cross-linked HA hydrogel (HA-XL, see below) expected to extend the duration of IOP reduction. In this second cohort, seven eyes received 50 µl of the HA-XL formulation injected into the SCS, two eyes received a sham injection of 50 µl of HBSS and one eye was injected with 50 µl of the commercial HA formulation. In this experiment, the HA-SH and PEGDA were mixed for 15 min before injection to initiate the crosslinking reaction and then injected into the SCS where the crosslinking was completed *in situ* in the eye. The eye receiving the commercial HA formulation was used only for clinical evaluations for 31 days because IOP returned to baseline well before that time. All injections in the second study were performed in the superotemporal quadrant of the eye. Animals in the second study were euthanized 121 days after injection, and the collected specimens were processed for histopathological examinations.

In the third study, four animals were used for measuring aqueous outflow facility. Each rabbit received 50 µl of the HA-XL formulation injected into the SCS in the superotemporal quadrant of a randomly chosen eye. The other eye served as a naive control without treatment. Animals in this cohort were euthanized 7 days after injection, and the outflow facility was measured in the enucleated eyes, as described below.

At the end of all studies, rabbits were euthanized under general anesthesia.

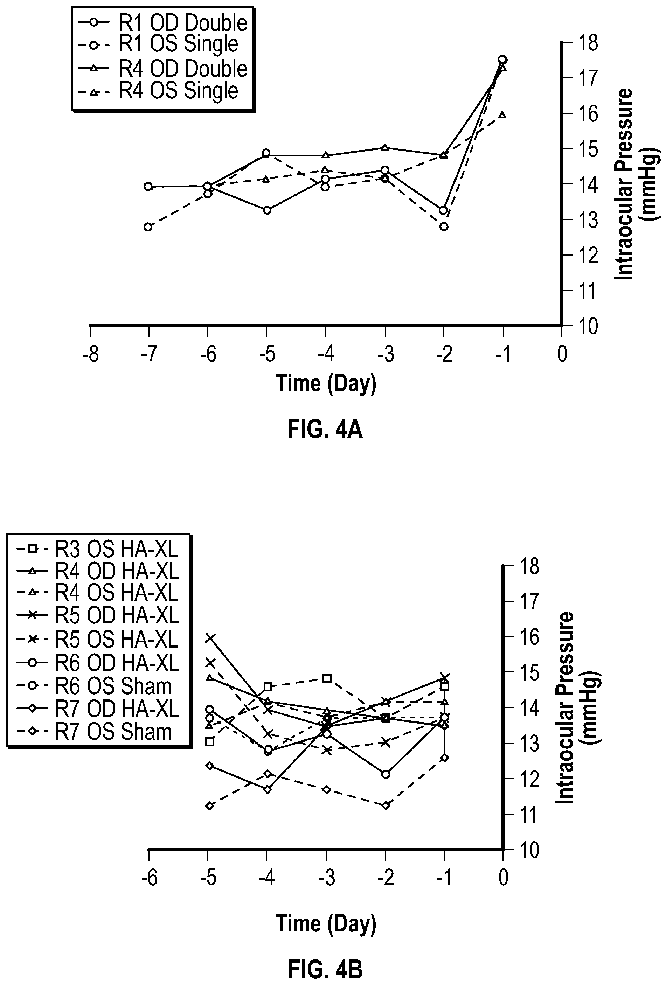

*Suprachoroidal injection:* General anesthesia was induced using an induction chamber with 5% isoflurane and a 400 ml/min oxygen flow rate for 7.5 min. Following induction, anesthesia was maintained with 2-3% isoflurane, which lead to transient ocular hypertension (18-30 mmHg). Before injection, the eye was irrigated with sterile saline, and then 5% of a povidone-iodine solution was instilled into the eyes for sterilization. After applying two drops of topical ophthalmic anesthetic, either hydrogel or Sham HBSS (i.e., HBSS without HA or PEGDA) was injected into the SCS of each eye with a hollow microneedle (shown in **FIG. 12**) at a location 2-3 mm posterior to the limbus. After the injection, successful SCS expansion was confirmed by ultrasound biomicroscopy, as described below, and an antibacterial ointment was applied to prevent secondary infection.
*Tonometry and clinical evaluations:* IOP was measured using a hand-held tonometer every day between 10 AM and 12 noon without the use of chemical agents or restraint. Recorded values were the average of five consecutive measurements. The baseline IOP value for each eye was taken as the average over five to seven days before the SCS injection for each animal within each treatment group. The measurement of the baseline IOP for each animal and each time point is shown in **FIGS. 4A-4B****,** wherein IOP variation of the baseline measurement for the first study is illustrated in **FIG. 4A** and the second study in **FIG. 4B** before the injection. The x-axes show days before hydrogel injection. After the baseline IOP measurement, each eye received a commercial hyaluronic acid (HA) hydrogel at one site (Single group) or two sites (Double group), an *in situ*-forming HA hydrogel (HA-XL group), or Hanks' Balanced Salt Solution (Sham group) at one site. Abbreviations- R: Rabbit identification number, OD: Right eye, OS: Left eye. R3 OD receiving the commercial HA formulation was used only for clinical evaluations. Data points represent individual measurements on the indicated rabbit.

Tonometric IOP measurements (IOPₜₒₙ) were converted to actual IOP (IOP_{act}) using a relationship obtained from a calibration study previously performed (J.J. Chae, M.R. Prausnitz, C.R. Ethier, J Am Assoc Lab Anim Sci, 60(1):91-95 (2021): IOP_{actual} = 0.8784 IOP_{tonometric} - 1.26 (IOP in units of mmHg). Briefly, a 25-gauge needle was inserted into the anterior chamber of five fresh *ex vivo* rabbit eyes. A reservoir was connected to the needle containing a balanced salt solution that was elevated to different heights to set IOP. The measurements by tonometry were produced as IOP was set at levels from 5 - 20 mmHg in increments of 5 mmHg, which were used for obtaining a calibration curve.

Clinical evaluations were performed using three ophthalmic instruments under general anesthesia by a veterinary ophthalmologist who prepared materials, preformed the injection, and measured the IOP of rabbits. For pre-anesthesia, the animal received a mixture of ketamine (10 mg/kg) and xylazine (2 mg/kg), after which anesthesia was maintained with 1.5 - 3.0% isoflurane. First, an ultrasound biomicroscope was used to image the SCS and surrounding ocular tissue structures. Serial images were acquired 10 min and 7, 14, 31, 42, 60, 91, and 120 days after the SCS injection. After attaching a sterile probe cover, the ultrasound probe was placed over the injection site. The maximum eye wall thickness (i.e., from the external conjunctival surface to the internal limiting membrane of the retina, including hydrogel in the SCS) at the injected site was calculated by averaging measurements obtained from three different images per time point using software bundled with the ultrasound imaging instrument. Baseline values of eye wall thickness were determined from images acquired from the non-injected equivalent area (i.e., 3 mm posterior to the limbus from the inferonasal quadrant of the eye).

Second, conventional examinations of the anterior eye were conducted using a slit-lamp with a standard table at 7, 14, and 31 days after the suprachoroidal injection. Third, the posterior eye was examined by instilling two drops of tropicamide and phenylephrine at 5-min intervals to dilate the pupil. Fundus images were obtained using a fundus scope attached to a 130° lens 7 days before and 7, 31, and 120 days after the SCS injection in a dark room.

Finally, photographs of the external features of the eyes were taken 1 h, and 3, 7, 14, and 31 days after the injection.

*Histopathological examination:* Eyes from the second study were enucleated, fixed with Davidson's solution and 10% formalin, dehydrated, and embedded in paraffin. Histological sections were cut using a rotary microtome and stained with either hematoxylin and eosin (H&E), using a standard procedure. The stained slides were imaged by brightfield light microscopy. More than four images for each group were evaluated by an ophthalmic pathologist masked as to treatment protocol.

*Outflow facility measurement:* Four pairs of eyes from the third study were enucleated 7 days after injection and mounted on eye holders in an iPerfusion system, which comprises an actuated pressure reservoir, a pressure transducer and a thermal flow meter. Enucleated eyes were cannulated with a 27-gauge 0.5-inch length sterile needle and perfused under a steady pressure of 11 mmHg for 30 min with DBG solution (1× Dulbecco's phosphate-buffered saline with 5.5 mM glucose). Ten sequential pressure steps of 7, 9, 11, 13.5, 16, 18.5, 21, 16, 12, and 8 mmHg were then applied to eyes by adjusting the height of the actuated reservoir. Flow rate (Q) and pressure (P) were recorded continuously until steady-state was reached, defined as the slope of the measured flow rate vs. time being less than 40 nl/min/min for 1 minute. Here, the slope was calculated from the change in recorded flow rate over a 300-second window, divided by 300 seconds. After the rejection of outlier steps, the outflow facility was calculated from fitting flow-pressure data at 9, 11, 13.5, 16, 18.5, and 21 mmHg using a power law. Because outflow facility is log-normally distributed, the variability of the data using a multiplicative standard deviation is reported, denoted by ^{×}/ (See J.M. Sherwood, E. Reina-Torres, J.A. Bertrand, B. Rowe, D.R. Overby, PLoS One 2016, 11, e0150694).

*Statistical analysis and data presentation:* All values are presented as mean ± standard deviation (S.D.). The first and second studies were statistically analyzed to evaluate the efficacy of each treatment. The number of samples in the HA-XL group was seven, and that of the other groups was two. Mean and 95% confidence intervals for baseline IOP values were calculated by combining all baseline measurements (across animals and days) within each treatment group. Least squares linear regression was used to model IOP vs. time post-injection in GraphPad Prism software (version 8.0.0 for Windows, GraphPad Software, San Diego, CA). For example, in the HA-XL group, linear regression was performed using the 7 eyes in that group, comprising 121 data points at different time points for each eye, meaning that the mean and 95% confidence bands calculated for this group were derived from 847 data points. The null hypothesis for the analysis of regressions was that one regression line adequately fit all treatment groups, with the threshold significance for the rejection of this hypothesis taken as *p = 0.05.* We found evidence to reject this hypothesis using GraphPad's extra sum-of-squares F test (*p < 0.0001*), substantiating the use of individual regression lines for each group. Mean and 95% confidence intervals for baseline IOP were compared to the mean and 95% confidence bands of the relevant treatment group's regression line. The time points at which statistically significant differences in IOP existed were determined using the intersection of 95% confidence intervals of the baseline IOP and 95% confidence bands of the treatment group IOP. It is an important distinction that the "95% confidence interval" is an indication of the certainty associated with the mean of the baseline values while the "95% confidence band" is an indication of the certainty associated with the location of the post-injection regression line. The intersection of 95% confidence intervals (for baseline means) or bands (for regression lines) is known to correspond with an approximate p-value of *p = 0.01*^{[45]} for the test of differences in parameters compared and was taken as the threshold for statistical significance for 1) comparison of values of two regression lines at every time point or 2) comparisons between a regression line value at every time point and the respective baseline mean. The time points at which significant differences occurred for compared values are reported.

In addition to comparisons of each treatment group's regression line to baseline IOP, Delta IOP was computed as the post-injection IOP minus that eye's baseline average IOP, regressed Delta IOP on time post-injection, and compared regression lines of between treatment groups and the sham group. Additionally, regression lines were compared between the Single and Double HA injection groups. The 95% confidence bands were calculated for each regression line, and statistically significant differences were determined using the intersection of 95% confidence bands between compared regression lines, as previously stated. Mean values were calculated from the regression line for each treatment group.

The normality of residuals for regression lines was tested using the D'Agostino-Pearson omnibus (K2) test, and the homoscedasticity of residuals was assessed using the test for homoscedasticity function in GraphPad.

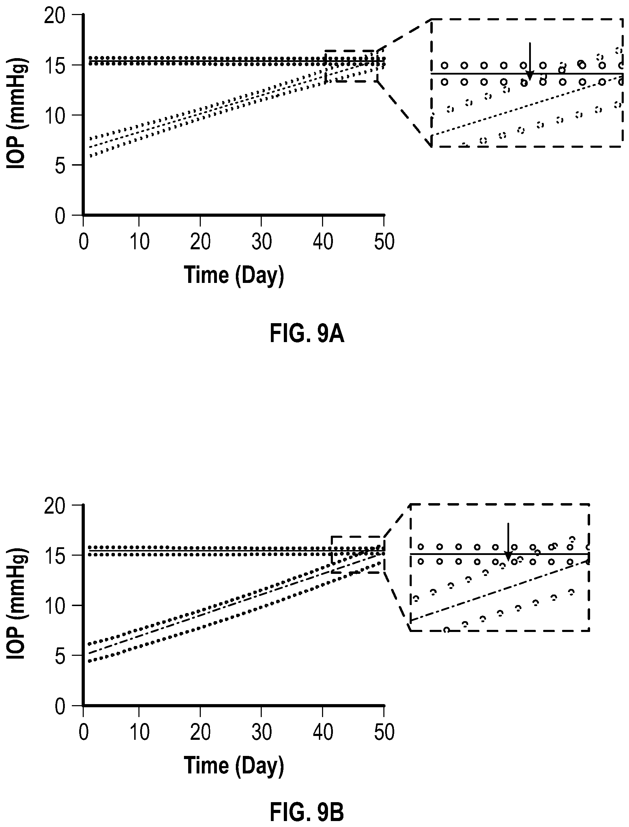

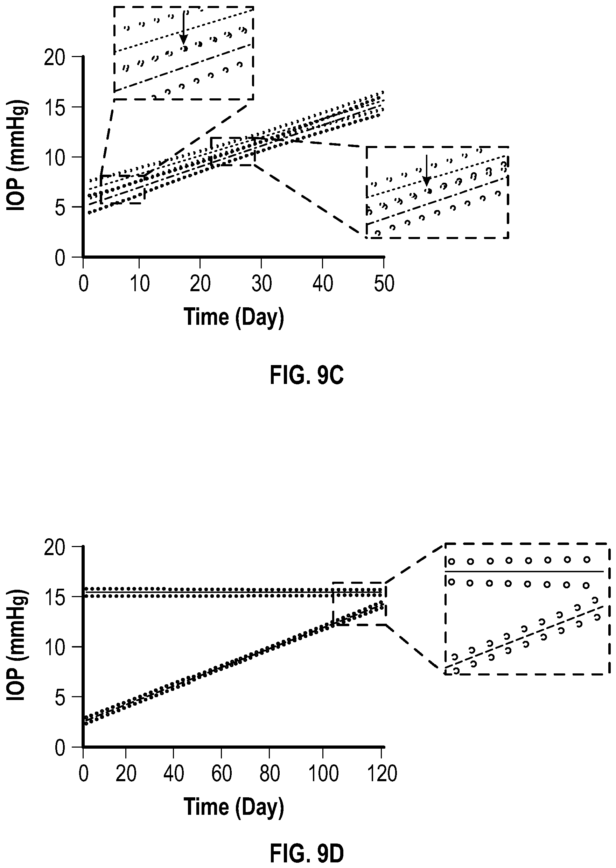

In **FIGS. 9A-9D****,** regression lines (solid lines) with 95% confidence bands (dotted lines) are plotted as IOP versus time post-injection. Insets show the intersection (indicated with arrows) of the 95% confidence bands. These bands were not overlapping through Day 43 for single HA compared to Sham (**FIG. 9A**) and Day 45 for Double HA compared to Sham (**FIG. 9B**). For Single HA compared to Double HA, the confidence bands were not overlapping from Day 8 through Day 28 (**FIG. 9C**). Confidence bands of the HA-XL treated eyes and Sham eyes did not overlap throughout the entire duration of the study (**FIG. 9D**). Results are from seven eyes (HA-XL group) or two eyes per group (Sham, single HA, and double HA groups). The significance of these graphs is that they evidence enablement of reduced IOP for 4 months.

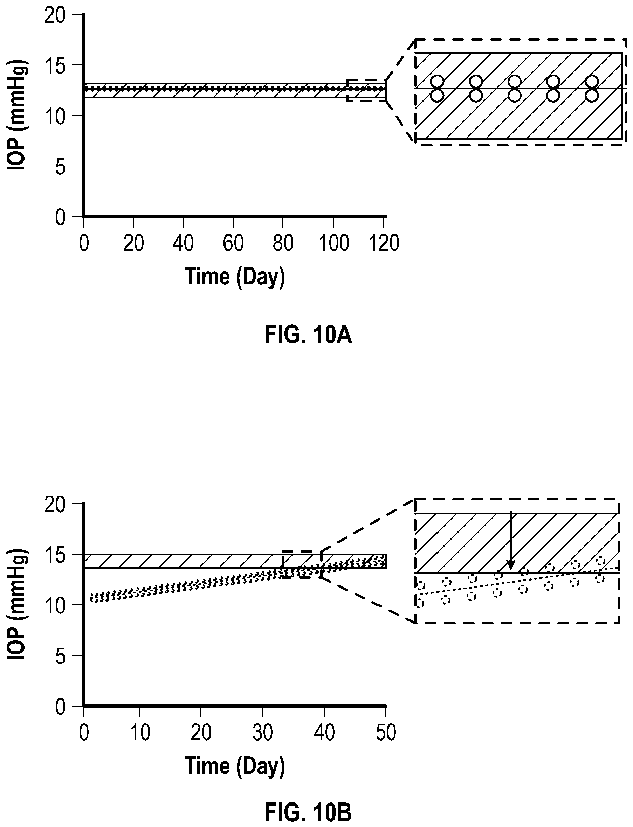

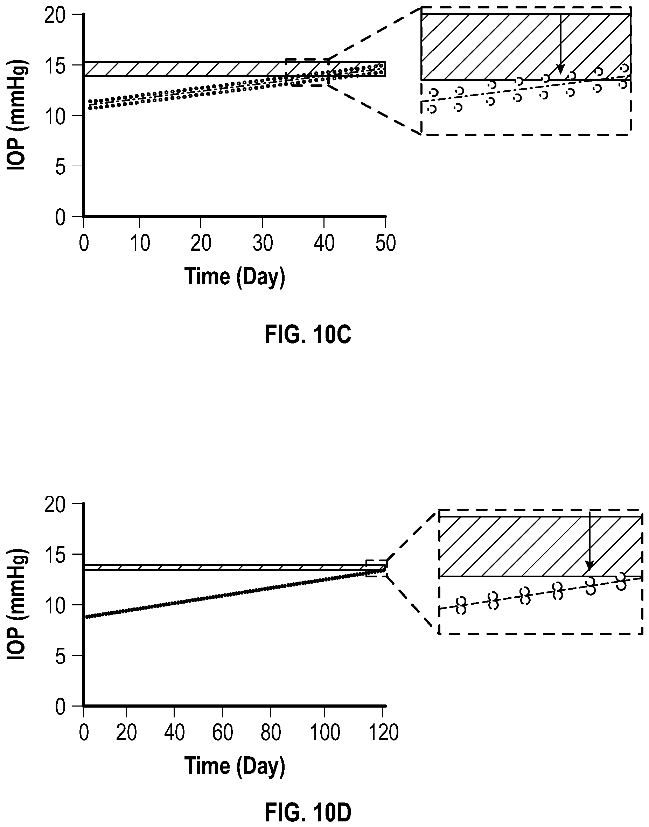

In **FIGS. 10A-10D****,** IOP post-injection was analyzed using linear regression and compared to baseline IOP. Regression lines (solid line) with 95% confidence bands (dotted lines) are plotted in addition to 95% confidence intervals (gray shading) for the respective baseline IOP values. Insets show the intersection (indicated with arrows) of the 95% baseline confidence interval with 95% post-injection confidence bands. These intervals/bands intersected for all time points in the Sham group (**FIG. 10A**), did not intersect through Day 35 for Single HA (**FIG.10B**), did not intersect through Day 35 for Double HA (**FIG. 10C**), and did not intersect through Day 119 for the HA-XL group (**FIG. 10D**). Results are from seven eyes (HA-XL group) or two eyes per group (Sham, single HA, and double HA groups).

### Example 1: SCS injection of commercial HA formulation lowered IOP for 35 days compared to baseline

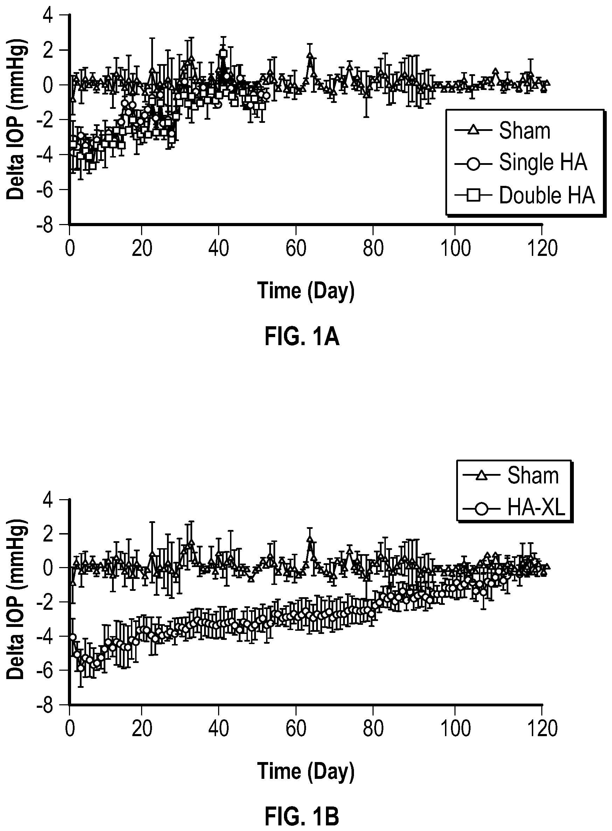

A commercial HA hydrogel developed as a skin filler to treat wrinkles was used. Using a normotensive rabbit model, it was observed that a single injection of 50 µl of this gel into the SCS led to an initial IOP reduction of approximately 4 mmHg (**FIG. 1A**). Over time, IOP steadily approached its baseline value. Analysis by linear regression showed that the gel-treated eyes had significantly lower IOP compared to their pre-injected values for 35 days (*p* <0.01). Additionally, the Delta IOP for treated eyes (current IOP minus baseline IOP) was significantly lower than that of the sham-treated eyes for 43 days (*p* < 0.01).

It was tested whether the extent or duration of IOP reduction might be improved by injecting the HA at two separate locations in each eye. The total volume of HA injected was held constant, so that 25 µl of HA was injected at each of the two injection sites in the double-injected eyes, whereas 50 µl of HA was injected in the single-injection eyes described above. Similar to the situation with a single injection, IOP after double HA injection dropped by approximately 4 mmHg right after injection, and then steadily increased to baseline values. Analysis by linear regression showed that IOP in the double-injected eyes was significantly lower than pre-injection IOP values for 35 days (*p* < 0.01). Additionally, the Delta IOP for the double-injected eyes remained significantly lower than that of the sham group for 45 days (*p* < 0.01). These findings further confirm that SCS injection of gel can reduce IOP for an extended period of time. When comparing the single-injected to the double-injected eyes, it was found that there were significant differences in IOP between days 8 and day 28 *(p* < 0.01), although the magnitude of this difference was small (< 0.6 mmHg) and thus likely clinically unremarkable.

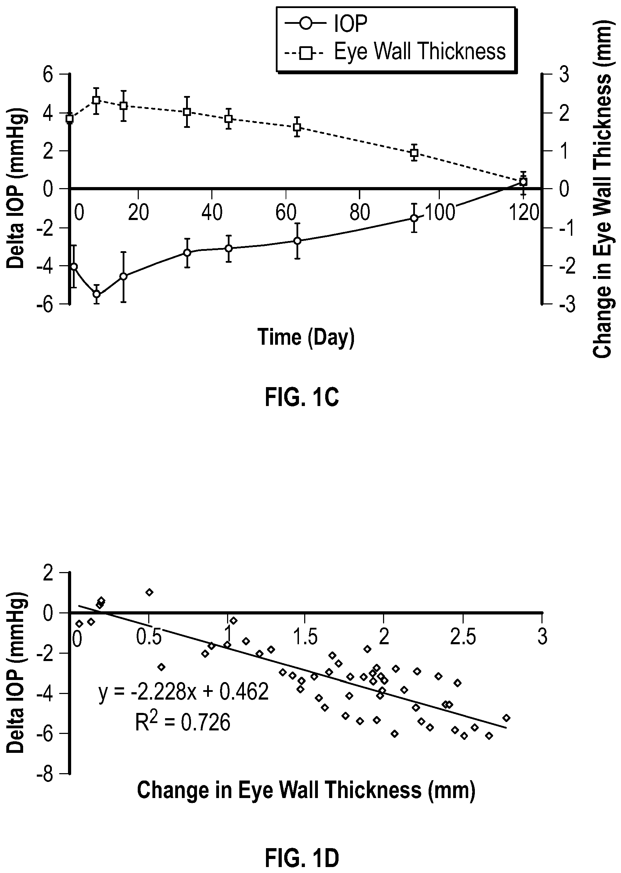

Overall, the effect of a suprachoroidal hydrogel injection on intraocular pressure (IOP) in the normotensive rabbit eye is shown in **FIGS. 1A-1D****.** In **FIG. 1A** a commercial hyaluronic acid (HA) hydrogel was injected into the suprachoroidal space (SCS) of the rabbit eye at one site (the superolateral quadrant, single HA group) or two sites (both the superolateral and superonasal quadrants, double HA group), or Hanks' Balanced Salt Solution (Sham) was injected into the SCS of the rabbit eye at one side (the superolateral quadrant). Change in IOP compared to pre-injection baseline values (Delta IOP) is shown. **FIG. 1B** shows delta IOP after an in situ-forming HA hydrogel (HA-XL) or Sham was injected into the SCS in the rabbit eye. **FIG. 1C** shows delta IOP after HA-XL injection compared to the mean change in posterior eye wall thickness in the eye over time. Change in eye wall thickness is interpreted as the degree of SCS expansion. **FIG. 1D** is a cross-plot of change in posterior eye wall thickness vs. Delta IOP (data from the **FIG. 1C**). Results are presented as mean ± standard deviation from seven eyes (HA-XL group), two eyes per group (Sham, single HA, and double HA groups) or seven eyes per group (eye wall thickness). The same data for the sham-injected eyes are shown in **FIGS. 1A-1B****.**

### Example 2: An optimized cross-linked HA formulation better-resisted degradation

To further extend IOP reduction, new HA hydrogel formulations designed to resist degradation, with the goal of sustaining SCS expansion and IOP reduction were created. Thiol groups were added to the HA (HA-SH) and co-formulated with polyethylene glycol diacrylate (PEGDA) as a cross-linker. As a baseline measurement, the degradation rate of the commercial HA-based hydrogel was assayed, which contained 2.4% (w/v) HA with 1,4-butanediol diglycidyl ether cross-linker, and found that it dissolved within 2 days in a hyaluronidase solution *in vitro.* HA solutions without a crosslinking agent were also prepared; it was found that gels composed of 4% (w/v) HA or 8% (w/v) HA dissolved within 2 or 4 days, respectively, when incubated with hyaluronidase.

In contrast, cross-linked gels created using HA-SH and PEGDA (i.e., HA-XL) persisted for more than one month in the hyaluronidase solution. Complete degradation occurred in the less-cross-linked gel after 33 days (5% (w/v) PEGDA) and in the more-cross-linked gel after 40 days (9% (w/v) PEGDA). These data suggest that the HA-XL formulations will persist in the eye for much longer than the commercial HA formulation, and thereby provide prolonged IOP reduction.

### Example 3: SCS injection of cross-linked HA formulation lowered IOP for four months compared to baseline

A single SCS injection of the more-cross-linked HA-XL gel formulation in normotensive rabbits led to an IOP reduction of approximately 4 mmHg immediately after injection, which then extended to an IOP drop of ~6 mmHg over the following 3 days (**FIG. 1B**). IOP then steadily increased over time and approached baseline levels. Statistical analysis showed that mean IOP in the eyes receiving HA-XL gel injection, as predicted by linear regression, was significantly lower than pre-injection IOP values for 119 days after injection (*p* < 0.01). Additionally, Delta IOP was significantly lower than in the sham group throughout the 121 day measurement period (*p* < 0.01). These data indicate that the injection of the HA-XL gel formulation into the SCS was able to produce an IOP reduction for 4 months.

### Example 4: IOP reduction correlated with SCS expansion

It was found that reduced IOP is associated with the expansion of the SCS, and the greater SCS expansion correlates with greater IOP reduction. Consistent with this, it was shown that when IOP reduction was greatest (i.e., right after SCS injection of HA-XL gel), expansion of the SCS measured by ultrasound biomicroscopy was also greatest (**FIG. 1C**). The initial increase in IOP reduction from ~4 mmHg to ~6 mmHg during the first week after injection corresponded to an increase in eye wall thickness of ~4 mm to ~5 mm. The measured increase in eye wall thickness is interpreted as an increase in SCS thickness since the tissues of the eye are not expected to expand (or contract) due to HA-XL injection, but the SCS is expected to widen. After the first week, as IOP reduction steadily decreased, SCS expansion similarly steadily decreased until 120 days, when there was no longer a significant IOP reduction, and the SCS appeared to be fully collapsed. Statistical analysis showed a quantitative correlation between the amount of IOP reduction and the change in eye wall thickness (linear regression, R² = 0.726) (**FIG. 1D**). According to this correlation, each increase in eye wall thickness of ~500 µm corresponded to a reduction of ~1 mmHg IOP in normotensive rabbits.

Expansion of the SCS after injection of HA was examined in greater detail by ultrasound biomicroscopy. Imaging showed an expanded anterior SCS immediately behind the ciliary body after SCS injection. Sham injection of Hanks' Balanced Salt Solution (HBSS) caused only minor SCS expansion 10 minutes after injection and was no longer evident after that. After injection of commercial HA gel, the SCS also expanded but collapsed almost to baseline thickness within 14 days, with no expansion visible 31 days after injection.

After injection of HA-XL gel, SCS expansion was evident 10 min after injection and grew in size during the first week, which was attributed to initial gel swelling as it imbibed water from neighboring tissues. The SCS then remained expanded for at least 91 days (although less expanded at later times) and appeared fully collapsed by 120 days after injection. SCS expansion appeared to be highly localized to the anterior SCS, extending from the anterior end of the SCS adjacent to the ciliary body to as far as 4 - 5 mm posteriorly. The expansion of the SCS appeared up to ~3 mm thick at its peak. These findings support that IOP reduction is correlated with SCS expansion.

### Example 5: SCS injection of cross-linked HA appears safe

During the course of the experiments, the rabbit eyes underwent periodic clinical examinations to assess the safety of the SCS injection of HA gels. After the sham injection of HBSS or injection of commercial HA, eyes did not show any remarkable changes in their external features immediately after injection or over time. After HA-XL injection, eyes appeared normal immediately after the injection, but by day 3 after injection, eyes presented with mild to moderate surface hyperemia at the site of injection, indicating localized irritation. The hyperemia lessened over time and later fully resolved within 1 - 2 weeks. Because the commercial HA gel did not have this effect, we attribute the irritation to the PEGDA crosslinking agent at the amounts used, extent of pre-injection crosslinking, and other parameters. All other external features in the clinical exam appeared normal.

Slit-lamp biomicroscopy exams appeared normal during all exams for the Sham, HA gel, and HA-XL gel formulations. There was no evidence of flare (indicating inflammation in the anterior segment), which suggested that the hyperemia seen in the clinical exam was localized to the ocular surface and did not induce uveitis. Also, no significant structural abnormality was observed by ultrasound biomicroscopy in any group of animals.

Fundoscopy was also generally unremarkable. Retinas and posterior structure of the rabbit eyes were clearly visible, and there were no floating particles or layers in the posterior eye that suggested evidence of retinal detachment, tears, hemorrhage, hydrogel leaking, or vitreous hemorrhage. However, the peripheral retina of eyes receiving either HA or HA-XL gel appeared to be "bulging out." This retinal deformation was first seen one week after injection and was found to resolve over time. This bulging appearance was attributed to the presence of gel expanding the SCS, which provides further support that the injection of gel into the SCS expands SCS thickness.

At the end of the study, i.e., 121 days after injection, the eyes were enucleated and subjected to histopathological examination. Tissues in sham-injected eyes did not show any significant abnormalities. However, evidence of local (non-expulsive) hemorrhage was found in the rabbit eye that had received HA gel. Near the injection site of eyes receiving HA-XL gel, eyes exhibited mild immune cell filtration, minimal to moderate fibrotic formation, and an enlarged suprachoroidal space, which suggests that the extended presence of HA-XL gel in the SCS generated a local immune response.

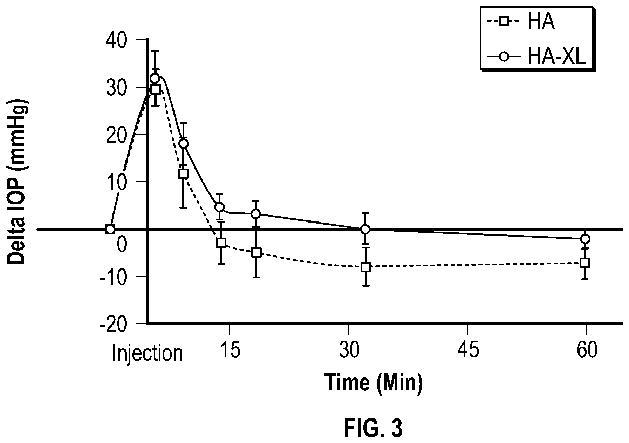

Immediately after injection of Sham, HA, or HA-XL formulations, there was a transient increase of IOP, which subsequently dropped to baseline within one hour (FIG. 3). This behavior is consistent with prior studies of SCS injection of other formulations and is believed to be due to the increase of intraocular volume caused by the introduction of fluid into the eye (i.e., in this case, 50 µl of fluid) followed by a return to baseline pressure as the fluid is cleared from the eye (e.g., via choroidal vessels and/or across the sclera).(See B. Chiang, K. Wang, C.R. Ethier, M.R. Prausnitz, Invest Ophthalmol Vis Sci 2017, 58, 545.) This is also known to occur routinely after intravitreal injection, where it is generally well tolerated.

### Example 6: IOP reduction is not due to changes in conventional outflow

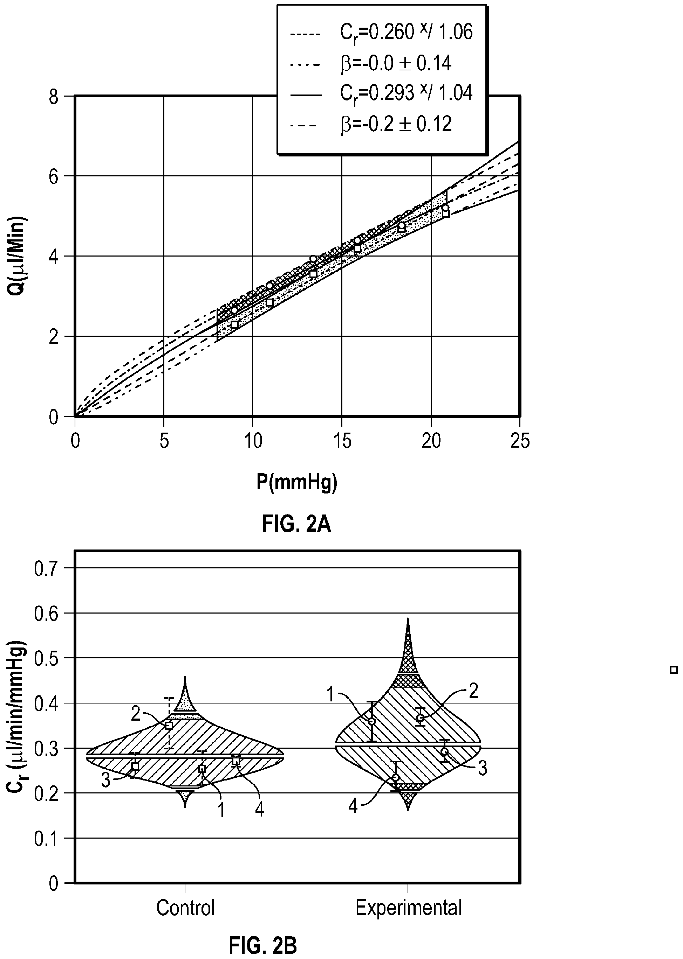

To further understand the effects of injected HA hydrogel on aqueous humor dynamics, conventional outflow facility was measured using iPerfusion, an *ex vivo* method to determine the resistance to fluid flow out of the eye as a function of IOP (**FIGS. 2A-2D**). One week after injecting HA-XL gel into the SCS of rabbits, the animals were euthanized and outflow facility was measured in enucleated eyes. No significant difference (*p* = 0.41) of outflow facility was observed between control (0.28 ^{×}/ 1.30 µl/min/mmHg) and HA-XL hydrogel-injected eyes (0.31 ^{×}/ 1.40 µl/min/mmHg), which means that hydrogel injection into the SCS did not appear to significantly affect aqueous outflow dynamics Since this technique specifically measures conventional outflow facility through the trabecular meshwork, this finding supports that increased uveoscleral outflow via the unconventional pathway was responsible for lower IOP in hydrogel-treated eyes.

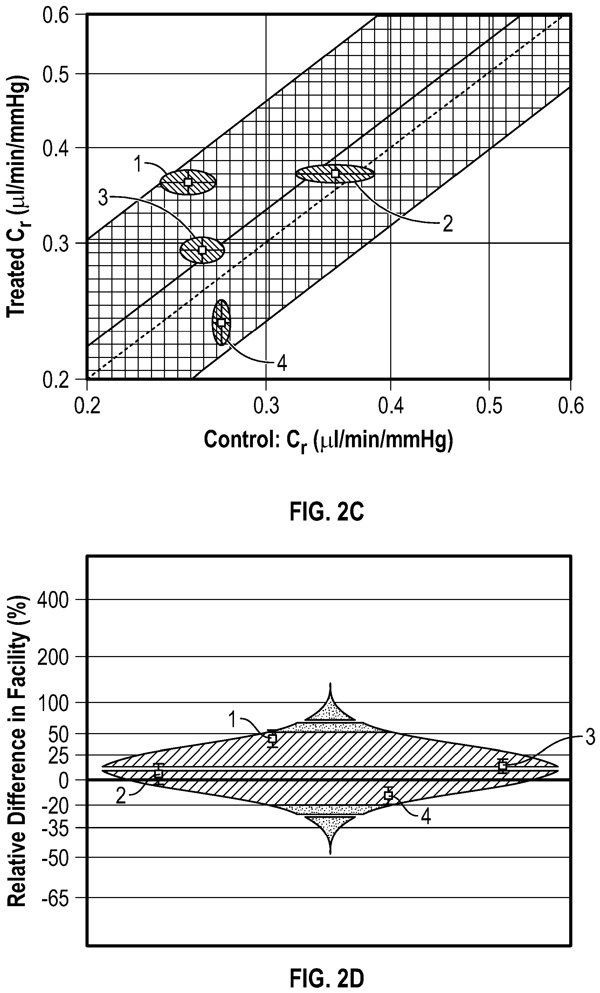

More specifically, **FIGS. 2A-2D** show measurements of aqueous outflow facility to identify the mechanism of IOP lowering after injection of *in situ*-forming hydrogel (HA-XL). **FIG. 2A** is a representative flow-pressure (Q-P) trace for an eye receiving HA-XL and the contralateral naive control eye. Data points are fitted with an existing relationship (solid lines) with associated 95% confidence limits (shaded region), and used to obtain a conventional outflow facility, *Cᵣ*, at a reference pressure of 8 mmHg. In **FIG. 2B** the data is presented in a "cello plot," showing the distribution of reference facilities in eyes receiving HA-XL ( n = 4) and naive contralateral control eyes (n = 4). Each data point shows the reference facility for one eye, with the error bars showing 95% confidence intervals. Shaded regions show the best estimates of the sample distributions, with the geometric mean and two-sigma shown by the thick and thin horizontal lines, respectively. Dark central bands show the 95% confidence interval on the mean. **FIG. 2C** is a "unity plot" for reference outflow facility, in which facility in the treated eye is cross-plotted against the facility in the contralateral control eye. Each point represents one animal, with ellipses indicating 95% confidence intervals from the regression fitting. **FIG. 2D** shows that the HA-XL injection leads to a mild (10%) increase in reference outflow facility, which is not statistically significant (*p* = 0.41, paired t-test on log-transformed data with threshold *p* = 0.05). The plotted quantity is the percentage difference between contralateral eyes, and the plot is otherwise interpreted as in **FIG. 2B****.** **FIGS. 2B-2D** show the same data plotted in different ways.

## Claims

1. A composition for use in lowering intraocular pressure (IOP) in a patient, the composition comprising:
a biocompatible fluid formulation which comprises a hydrogel material and does not include an active pharmaceutical ingredient,
**characterized in that**
before and/or following introduction of the biocompatible fluid formulation into a suprachoroidal space (SCS) of an eye of the patient, the hydrogel material is cross-linked such that the composition is able to remain within the SCS for at least 30 days.

2. The composition of claim 1, wherein the biocompatible fluid formulation is a shear-thinning fluid.

3. The composition of claim 1, wherein the biocompatible fluid formulation is configured to undergo in-situ solidification.

4. The composition of any one of claims 1 to 3, wherein the hydrogel material is configured to crosslink or further crosslink in vivo.

5. The composition of claim 4, wherein the hydrogel material comprises hyaluronic acid.

6. The composition of claim 5, wherein the hyaluronic acid comprises added thiol groups.

7. The composition of claim 5, wherein hyaluronic acid is co-formulated with polyethylene glycol diacrylate (PEGDA).

8. The composition of any one of claims 1 to 7, wherein the composition is configured to biodegrade and clear from the patient's eye.

9. The composition of any one of claims 1 to 8, wherein the biocompatible fluid formulation comprises a biopolymer, a solvent, and optionally a pore-forming agent.

10. A composition for use according to any one of claims 1 to 9 wherein the patient is indicated to have or to be at risk of developing glaucoma and wherein the composition is injected
into a suprachoroidal space (SCS) of an eye of the patient.

11. The composition for use of claim 10, wherein the reduction in intraocular pressure is maintained for at least 60 days, preferably at least 90 days, and more preferably at least 120.

12. The composition for use of claim 10 or 11, wherein the injecting comprises:
inserting a hollow needle, preferably a microneedle, into the eye;
injecting a bolus of the composition in fluid form through the hollow needle and into the SCS of the eye; and then
removing the hollow needle from the eye,
wherein the composition expands the SCS and solidifies within the SCS.

13. The composition for use of claim 12, wherein the composition solidifies within the SCS by gelation.

14. The composition for use of claim 13, wherein the gelation is facilitated by a change in pH, ionic strength, or by contact with biological fluids within the SCS.

15. The composition for use of claim 10 or 11, wherein the injecting comprises:
inserting a hollow needle, preferably a microneedle, into the eye;
injecting a bolus of the composition in fluid form through the hollow needle and into the SCS of the eye; and then
removing the hollow needle from the eye,
wherein the composition comprises a crosslinked hydrogel before the injecting.

## Patentansprüche

1. Zusammensetzung zur Senkung des Augeninnendrucks (*intraocular pressure*, IOP) bei einem Patienten, wobei die Zusammensetzung umfasst:
eine biokompatible Flüssigkeitsformulierung, die ein Hydrogelmaterial umfasst und keinen pharmazeutischen Wirkstoff enthält,
**dadurch gekennzeichnet, dass**
vor und/oder nach der Einführung der biokompatiblen Flüssigkeitsformulierung in einen suprachoroidalen Raum (*suprachoroidal space*, SCS) eines Auges des Patienten das Hydrogelmaterial so vernetzt wird, dass die Zusammensetzung mindestens 30 Tage lang im SCS verbleiben kann.

2. Zusammensetzung nach Anspruch 1, wobei die biokompatible Flüssigkeitsformulierung eine scherverdünnende Flüssigkeit ist.

3. Zusammensetzung nach Anspruch 1, wobei die biokompatible Flüssigkeitsformulierung so konfiguriert ist, dass sie eine In-situ-Verfestigung durchläuft.

4. Zusammensetzung nach einem der Ansprüche 1 bis 3, wobei das Hydrogelmaterial so konfiguriert ist, dass es in vivo vernetzt oder weiter vernetzt wird.

5. Zusammensetzung nach Anspruch 4, wobei das Hydrogelmaterial Hyaluronsäure umfasst.

6. Zusammensetzung nach Anspruch 5, wobei die Hyaluronsäure zugesetzte Thiolgruppen umfasst.

7. Zusammensetzung nach Anspruch 5, wobei die Hyaluronsäure mit Polyethylenglykoldiacrylat (PEGDA) co-formuliert ist.

8. Zusammensetzung nach einem der Ansprüche 1 bis 7, wobei die Zusammensetzung so konfiguriert ist, dass sie biologisch abbaubar ist und aus dem Auge des Patienten ausgeschieden wird.

9. Zusammensetzung nach einem der Ansprüche 1 bis 8, wobei die biokompatible Flüssigkeitsformulierung ein Biopolymer, ein Lösungsmittel und optional ein porenbildendes Mittel umfasst.

10. Zusammensetzung zur Verwendung gemäß einem der Ansprüche 1 bis 9, wobei bei dem Patienten ein Glaukom festgestellt wurde oder das Risiko besteht, ein Glaukom zu entwickeln, und wobei die Zusammensetzung in den suprachoroidalen Raum (SCS) eines Auges des Patienten injiziert wird.

11. Zusammensetzung zur Verwendung nach Anspruch 10, wobei die Senkung des Augeninnendrucks für mindestens 60 Tage, vorzugsweise mindestens 90 Tage und noch bevorzugter mindestens 120 Tage aufrechterhalten wird.

12. Zusammensetzung zur Verwendung nach Anspruch 10 oder 11, wobei die Injektion umfasst:
das Einführen einer Hohlnadel, vorzugsweise einer Mikronadel, in das Auge;
das Injizieren eines Bolus der Zusammensetzung in flüssiger Form durch die Hohlnadel in den SCS des Auges; und anschließend
das Entfernen der Hohlnadel aus dem Auge,
wobei die Zusammensetzung den SCS ausdehnt und innerhalb des SCS erstarrt.

13. Zusammensetzung zur Verwendung nach Anspruch 12, wobei die Zusammensetzung innerhalb des SCS durch Gelierung verfestigt.

14. Zusammensetzung zur Verwendung nach Anspruch 13, wobei die Gelierung durch eine Änderung des pH-Werts, der Ionenstärke oder durch Kontakt mit biologischen Flüssigkeiten innerhalb des SCS begünstigt wird.

15. Zusammensetzung zur Verwendung nach Anspruch 10 oder 11, wobei das Injizieren umfasst:
Einführen einer Hohlnadel, vorzugsweise einer Mikronadel, in das Auge;
Injizieren eines Bolus der Zusammensetzung in flüssiger Form durch die Hohlnadel in den SCS des Auges; und anschließend
Entfernen der Hohlnadel aus dem Auge,
wobei die Zusammensetzung vor dem Injizieren ein vernetztes Hydrogel umfasst.

## Revendications

1. Composition destinée à réduire la pression intraoculaire (*intraocular pressure*, IOP) chez un patient, ladite composition comprenant :
une formulation fluide biocompatible qui comprend un matériau hydrogel et ne contient pas d'ingrédient pharmaceutique actif,
**caractérisée en ce que**
avant et/ou après l'introduction de la formulation fluide biocompatible dans un espace suprachoroïdien (*suprachoroidal space*, SCS) d'un œil du patient, le matériau hydrogel est réticulé de telle sorte que la composition soit capable de rester dans le SCS pendant au moins 30 jours.

2. Composition selon la revendication 1, dans laquelle la formulation fluide biocompatible est un fluide à viscosité réduite sous cisaillement.

3. Composition selon la revendication 1, dans laquelle la formulation fluide biocompatible est configurée pour subir une solidification in situ.

4. Composition selon l'une quelconque des revendications 1 à 3, dans laquelle le matériau hydrogel est configuré pour se réticuler ou se réticuler davantage in vivo.

5. Composition selon la revendication 4, dans laquelle le matériau hydrogel comprend de l'acide hyaluronique.

6. Composition selon la revendication 5, dans laquelle l'acide hyaluronique comprend des groupes thiol ajoutés.

7. Composition selon la revendication 5, dans laquelle l'acide hyaluronique est coformulé avec du diacrylate de polyéthylène glycol (PEGDA).

8. Composition selon l'une quelconque des revendications 1 à 7, dans laquelle la composition est conçue pour se biodégrader et être éliminée de l'œil du patient.

9. Composition selon l'une quelconque des revendications 1 à 8, dans laquelle la formulation fluide biocompatible comprend un biopolymère, un solvant et, éventuellement, un agent porogène.

10. Composition destinée à être utilisée selon l'une quelconque des revendications 1 à 9, dans laquelle il est indiqué que le patient souffre d'un glaucome ou présente un risque de développer un glaucome, et dans laquelle la composition est injectée dans l'espace suprachoroïdien (SCS) d'un œil du patient.

11. Composition destinée à être utilisée selon la revendication 10, dans laquelle la réduction de la pression intraoculaire est maintenue pendant au moins 60 jours, de préférence au moins 90 jours, et plus préférablement au moins 120 jours.

12. Composition destinée à l'usage selon la revendication 10 ou 11, dans laquelle l'injection comprend :
l'insertion d'une aiguille creuse, de préférence d'une micro-aiguille, dans l'œil ;
l'injection d'un bolus de la composition sous forme fluide à travers l'aiguille creuse et dans l'espace suprachoroïdien (SCS) de l'œil ; puis
le retrait de l'aiguille creuse de l'œil,
dans laquelle la composition dilate l'espace suprachoroïdien (SCS) et se solidifie à l'intérieur de celui-ci.

13. Composition destinée à l'utilisation selon la revendication 12, dans laquelle la composition se solidifie au sein du SCS par gélification.

14. Composition destinée à l'utilisation selon la revendication 13, dans laquelle la gélification est facilitée par un changement de pH, de force ionique ou par contact avec des fluides biologiques au sein du SCS.

15. Composition selon la revendication 10 ou 11, dans laquelle l'injection comprend :
l'insertion d'une aiguille creuse, de préférence une micro-aiguille, dans l'œil ;
l'injection d'un bolus de la composition sous forme fluide à travers l'aiguille creuse et dans le SCS de l'œil ; puis
le retrait de l'aiguille creuse de l'œil,
la composition comprenant un hydrogel réticulé avant l'injection.
